Europäisches Patentamt

European Patent Office

Office européen des brevets

(11) Publication number: **0 385 385 B1**

## EUROPEAN PATENT SPECIFICATION

(45) Date of publication of patent specification: **27.09.95**

(51) Int. Cl.6: **A61K 38/00**

(21) Application number: **90103771.3**

(22) Date of filing: **27.02.90**

The file contains technical information submitted after the application was filed and not included in this specification

(54) **Human monocyte-machrophage-CSF preparations.**

(30) Priority: **28.02.89 JP 47594/89**
**04.04.89 JP 85612/89**
**30.03.89 JP 79100/89**
**12.05.89 JP 117372/89**
**12.06.89 JP 150087/89**

(43) Date of publication of application:
**05.09.90 Bulletin 90/36**

(45) Publication of the grant of the patent:
**27.09.95 Bulletin 95/39**

(84) Designated Contracting States:
**CH DE FR GB LI**

(56) References cited:
**WO-A-86/04607**
**WO-A-87/06954**
**WO-A-89/09060**

**BIOCHEMICAL ABSTRACTS vol. 87, accession no.**

(73) Proprietor: **MORINAGA MILK INDUSTRY CO., LTD.**
**33-1, Shiba 5-chome**
**Minato-ku,**
**Tokyo-to 108 (JP)**

(72) Inventor: **Kawashima, Takuji**
**3-18-5, Tsuchihashi,**
**Miyamae-ku**
**Kawasaki-shi,**
**Kanagawa (JP)**
Inventor: **Yanai, Nobuya**
**5-21-10-401, Minamidai,**
**Nokano-ku**
**Tokyo (JP)**
Inventor: **Yamada, Muneo**
**nr. 408 Sagamino-Sakura,**
**5-1-15, Higashihara**
**Zama-shi,**
**Kanagawa (JP)**
Inventor: **Yokota, Hajime**
**2-5-1-406, Shirogane,**
**Minato-ku**
**Tokyo (JP)**

EP 0 385 385 B1

89128162, abstract no. 62815, Biochemical Abstracts, Philadelphia, US; &Blood 1989, vol. 73, no. 1, pages 31-37; A. GANSER et al.: "Recombinant HumanGranulocyte-Macrophage Colony Stimulating Factor in Patients withMyelodysplastic Syndromes. A Phase I-II Trial"

BIOCHEMICAL ABSTRACTS vol. 87, accession no.8976573, abstract no. 40971, Biochemical Abstracts, Philadelphia, US; &J. Am. Med. Assoc. 1988, vol. 260, no. 22, pages 3297-3300; S.D. NIMER et al.:"Serum Cholesterol Lowering Activity of Granulocyte-Macrophage ColonyStimulating factor"

Motoyoshi et al Exp. Hematol. 1989 17;68-71

Wide Scope Syndrome 1987 Nihon rinsho-sha p465

Clinical Doctor 1988 vol 14

serum Lipid Chugai Igakusha 1981

Inventor: **Ujiie Kunio, Ikegami-Ryoh of Morinaga Nyugyo Kabushiki Kaisha,**
**2-10-16, Nakaikegami**
**Ohta-ku,**
**Tokyo (JP)**
Inventor: **Yoshida, Katsuo**
**1-37-15, Nangai**
**Higashiyamato-shi,**
**Tokyo (JP)**
Inventor: **Suzu, Shinya**
**1-6-5, Kokubunji-cho Eki-Higashi,**
**Shimotsuga-gun**
**Tochigi (JP)**
Inventor: **Takaku, Fumimaro**
**1-43-30, Asahigaoka,**
**Nerima-ku**
**Tokyo (JP)**
Inventor: **Motoyoshi, Kazuo**
**1238, Kamikomachi**
**Ohmiya-shi,**
**Saitama (JP)**
Inventor: **Yamada, Nobuhiro**
**3-33-9, Ogikubo,**
**Suginami-ku**
**Tokyo (JP)**

(74) Representative: **Baillie, Iain Cameron et al**
**c/o Ladas & Parry**
**Altheimer Eck 2**
**D-80331 München (DE)**

## Description

FIELD OF THE INVENTION

The present invention relates to novel medical indications for human monocyte-macrophage colony stimulating factor (hereinafter abbreviated as hM-CSF) preparations.

More particularly, the present invention relates to therapeutic preparations containing hM-CSF as the effective component for the treatment of Myelodyslastic syndrome (hereinafter abbreviated as MDS).

The present invention also relates to therapeutic preparations containing hM-CSF, particularly originating from human urine, as the effective component for the treatment of specific malignant tumors.

The present invention also relates to the therapeutic preparations mentioned above containing recombinant hM-CSF (hereinafter abbreviated as rhM-CSF) as an effective component.

BACKGROUND OF THE INVENTION

Colony stimulating factors (hereinafter abbreviated as CSFs) are known as substances which stimulate the proliferation and differentiation of haematopoietic stem cells, and they are categorized into the following four items:

(1) factors which act on the monocyte-macrophage lineage (M-CSFs),

(2) factors which act on the granulocyte-monocyte lineage (GM-CSFs),

(3) factors which act on the granulocyte lineage (G-CSFs), and

(4) factors which act on multipotential stem cells (multi-CSFs)

Human M-CSFs are already isolated and purified, and their protein structure and the genetic structure which participates in the production of M-CSFs or which encodes the amino acid sequence of M-CSFs has been described (cf: Japanese Unexamined Patent Application Gazette No. 64(1989) /22899).

The tumouricidal activity of a CSF is described by A. Samson-Johannes et al (cf: Journal of Immunology, Vol. 141, 3680-3686, No. 10, November 15 1988).

It is also known that an hM-CSF may stimulate a mature human monocytemacrophage to produce various cytokines (cf: K. Motoyoshi et al, Experimental Hematology, Vol. 17, 68-71, 1989); that a human urinary M-CSF is clinically effective on granulocytopenia (cf: K. Motoyoshi et al, Experimental Hematology, Vol, 14, 1069-1075, 1986), and that a human urinary M-CSF is clinically effective in the recovery of leukocyte and granulocyte numbers after bone marrow transplantation (cf: T. Masaoka et al, Bone Marrow Transplantation, Vol.3, 121-127, 1988). Therefore, human M-CSFs have been expected to be useful as a drug.

The safeness and scarcity of side effects in dosage of hM-cSF has also been clinically confirmed (cf: K. Motoyoshi et al, Immunobiology Vol 172, 205-212, 1986).

It is known that the monocyte-macrophage lineage is significantly related to hyperlipemia and arteriosclerosis.

PROBLEMS IN THE PRIOR ART

MDS (Myelodysplastic Syndrome) is a name assigned to the aggregation of symptoms wherein quantitative and qualitative disorders are found in one or more of the lineages of the hematopoietic system comprising erythrocyte, granulocytemonocyte, and platelet lineages. In this syndrome, various disorders are typically found widely in the bone marrow and the peripheral blood, for example, macrocytepemia, ringedsideroblastic anemia, megaloblastic anemia, neutropenia and thrombocytopenia, and chromosome aberration.

MDS is categorized into 5 types depending upon the patients' condition or the condition of the disease; refractory anemia (RA), RA with ringed sideroblastosis, RA with excess blasts (RAEB), chronic myelomonocytic leukemia (CMML), and RAEB in transformation. Although the symptoms are greatly different depending upon the type, any type of MDS is ultimately inverted into myelogeneous leukaemia, which is a mortal disease, after lapse of a few months or a few years. Although minimum Ara-C dosing therapy of $VD_3$ therapy has been clinically applied, therapeutic results are not satisfactory. Therefore establishment of an effective therapy has been earnestly desired.

However, the utilizability of hM-CSF against MDS has remained to be studied.

Therapeutic effects against malignant tumors have been improved by the development of chemotherapeutic preparations as well as by improvements in surgical techniques. Complete elimination of malignant tumors, however, has not been achieved, and relapses are often observed. It is also a problem that chemotherapeutic preparations have severe side effects on the various organs, and that dosage of chemotherapeutic preparation often causes heavy infectious diseases and/or organopathy. Consequently, bone marrow transplantation after heavy or strong chemotherapy has been tried for a radical cure. In bone marrow transplantation, bone marrow grafts from donors having a coincident or matching HLA type have been utilized, however the manifestation of grafts-versus-host ractions (GVHR) have been often observed. Therefore, homologous bone marrow transplantation is desirable. In the case of tumors of hematopoietic organs, the inclusion of malignant tumor cells in bone marrow cells to be transplanted (or in the grafts) is inevitable, thus purge of malignant tumor cells has been essential.

However, the utilizability of human urinary M-CSF against specified malignant tumors has remained to be studied.

Medical treatments against malignant tumors also include surgical treatment, chemotherapy and radiotherapy. Therapeutic effects against malignant tumors have been improved by developments of chemotherapeutic preparations as well as by improvements in surgical techniques. However, since there is no chemotherapeutic preparation which has a remarkable therapeutic efficacy but low side effects, there are upper limits of their dosages. Again, complete elimination of malignant tumors has not been achieved and relapses have been often observed. There are chemotherapeutic preparations which show serious toxicities against hematopoietic organs and various other organs resulting in organic troubles and/or heavy infectious diseases. For example, anticancer preparations containing platinum complex compounds have excellent anti-tumor activities (cf: B. Rosenberg et al, Nature 205, 698, 1965). It has been reported that cis-platinum diamine dichloride (cisplatin CDDP), which is one of the platinum complex preparations, has a strong anti-tumor activity and that it demonstrates a remarkable effect against malignant ureteral tumors and gynecological malignant tumors which had hitherto been difficult to cure by chemotherapy (cf: Merrin C.E., Cancer Treat. Rep., 63, 1579, 1979). It is a problem that these chemotherapeutic preparations have strong toxicity on kidneys and hematopoietic organs, and often result in renal insufficiency, thrombocytopenia, anemia and other serious side effects.

Hyperlipemia is known as a disease wherein serum levels of one or more of cholesterol, neutral fats and phospholipids are increased over the respective normal or reference values. The reference values for hyperlipemia for Japanese persons are settled as 220 mg of total cholesterol, 130 mg of neutral fats, and 250 mg of phospholipids per 100 mℓ of blood respectively. Hyperlipemia by itself it not a serious disease, however, when it is left as it is, it may result in arteriosclerosis, and it may be an inducement for angina pectoria or stenocardia and myocardial infarction. Thus it may result in serious consequences from a clinical view point. Although there are many drugs against hyperlipemia and arteriosclerosis, clinically probucol (cf: A. Watanabe et al, Arteriosclerosis in Japanese Vol. 11, No. 3597, 1983) and elastase (cf: S. Yoshimura, Arteriosclerosis in Japanese VOI 3, 223, 1975) have been mainly utilized. The functions of these drugs are to minimise adherence of cholesterol to vascular walls or to give washing out of cholesterol therefrom. However, there are limitations on their effects and there is no drug to achieve a radical cure of hyperlipemia.

SOLUTION OF THE PROBLEMS

Some of the inventors (F. Takau and K. Motoyoshi) conducted studies about the utilizability of hM-CSF as a therapeutic preparation against MDS. As the result, it has been found that recovery from the decrease or the disappearance of myeloblast cells (which are serious problems resulting from MDS), and an increase in the normal leukocyte and normal erythrocyte count can be achieved by the dosage of hM-CSF. The present invention is based on the discovery.

Some of the inventors (T. Kawashima, N. Yanai, M. Yamada, H. Yokota, K. Ujiie, K. Yoshida and S. Suzu) have conducted studies about the utilization of hM-CSF to kill and wound specific malignant tumor cells, and found that hM-CSF may augment the tumoricidal activity of the macrophage. The present invention is based on this discovery.

OBJECTS OF THE INVENTION

Therefore, it is an object of the present invention to provide specific hM-CSF preparations and specific uses therefor.

It is a particular object of the present invention to provide therapeutic preparation containing hM-CSF as an effective component against MDS.

It is a further object of the present invention to provide an anticancer preparation containing human urinary M-CSF as an effective component which has a remarkable anti-cancer activity against specific cancers and which has no severe side effects.

It is a still further object of the present invention to provide a regimen wherein the specific anticancer preparation containing hM-CSF as an effective component is dosed together with a specific antibody to malignant tumors.

It is a further object of the present invention to provide a therapeutic preparation containing rhM-CSF as an effective component, which is produced by mammalian cells transfected with the hM-CSF producing gene.

Human M-CSF used in the present invention may be prepared from human urine or a culture both in which hM-CSF producing cells or mammalian cells transfected with the hM-CSF-producing gene are cultivated.

Optionally there are also provided hM-CSF preparations in which surface active agents and/or proteins such as Tween® 80, human serum albumin or gelatine are contained as stabilizing agents for hM-CSF. Although, the effectiveness of other stabilizers other than those specifically enumerated has not yet been confirmed, it is anticipated that other surface active agents and other proteins originating from human beings will also be effective.

## DETAILED DESCRIPTION OF THE INVENTION

Human urinary M-CSF and rhM-CSF used in the present invention as hM-CSF was isolated, purified and freeze-dried in accordance with the known procedures described hereinafter.

The following references are acknowledged as prior art for the preparation of hM-CSF.

Japanese Unexamined Patent Application Gazette No.63(1988)-198700,

Japanese Unexamined Patent Application Gazette No.63(1988)-250400,

Japanese Unexamined Patent Application Gazette No.64(1989)-22899

[PREPARATION OF HUMAN URINARY M-CSF, #1] (cf: Japanese Unexamined Patent Application Gazette No. 63(0988)/198700)

From healthy men, 1,000 ℓ of human urine was collected, and its pH was adjusted to 8.5 to remove precipitate with a filter. The resultant filtrate was concentrated and demineralized using ultrafiltration membranes (by Amicon, H 10 × 50). The resultant concentrate was adjusted until its pH was 7.0, then pasturized in an air-tight container at 60°C for 10 hours. The pasturized liquid was centrifuged (5,000G × 30 min.) to remove precipitates, then the resultant supernatant was treated with a DEAE-cellulose column, which had been preliminarily equilibrated with 0.02 M phosphate buffer solution (ph 7.2), for adsorption. After washing the DEAE-cellulose with 0.02 M phosphate buffer solution (pH 7.2) containing 0.05 M sodium chloride, elution was carried out using 0.02 M phosphate buffer solution (pH 7.2) containing 0.25 M sodium chloride. The resultant eluate was concentrated with an ultrafiltration membrane (by Amicon, H 10 P 10) The thus obtained concentrate was subjected to gel filtration by Sephacryl® S-30 (trademark, by Pharmacia, 20 cm diameter × 80 cm hight) with a buffer solution (pH 7.2) containing 1 M ammonium sulfate. The 70,000 - 50,000 daltons fraction obtained from the gel filtration was applied to a phenyl-Sephalose® 4B column (trademark, by Pharmacia, 10cm diameter × 20 cm hight), which had been preliminarily equilibrated with the buffer solution containing ammonium sulfate, for adsorption, then eluted with the same buffer solution (pH 7.2) containing 0.5 M ammonium sulfate. The resultant eluate was concentrated with an ultrafiltration membrane (by Amicon, H 1 P 10) . The resultant concentrate was subjected to liquid chromatography (TSK-G3000SW, by Toso, 2 cm diameter × 60 cm hight) thereby obtaining the fraction of relative eluate quantity (Ve/Vo) 1.2 - 1.5. The resultant fraction was concentrated again. The thus obtained concentrate was subjected to reversed-phase high performance liquid chromatography (hereinafter abbreviated as RP-HPLC) using a Hi-Pore 214Tp column (by Biduck, 2.2 cm × 25 cm hight) with a 0 - 100% linear concentration gradient of acetonitrile containing 0.1 % trifluoroacetic acid. Thereby the hM-CSF fraction was collected. The collected hM-CSF fraction was freeze-dried, and 4mg of human urinary M-CSF was obtained.

[PREPARATION OF HUMAN M-CSF, #2] (cf: Japanese Unexamined Patent Application Gazette No. 64(1989)/22899).

Anti-hM-CSF antibody, which was collected from rabbits immunized with purified hM-CSF, was dyalized against 0.1 M phosphate buffer solution (pH7.0) to obtain 20 mg/mℓ antibody solution. To 100 g of Formyl-Cellulofine® (trademark, by Chisso Manuf.), which had been previously washed with distilled water and 0.1 M phosphate buffer solution, 200 mℓ of the antibody solution was added, and the resultant mixture was stirred at room temperature for adsorption, then 700 mg of sodium cyanoboron hydride was further added and stirred for 16 hours to thereby obtain antibody-bonded carriers wherein anti-hM-CSF antibody was bonded to Formyl-Cellulofine® (trademark, Chisso Manuf.). The resultant antibody-bonded carrier was washed with 0.2 M tris-HCℓ buffer solution, then 200 mℓ of the tris buffer solution containing 500 mg of sodium cyanoboron hydride was added and stirred for 4 hours at room temperature to deactivate unreacted formyl groups. The resultant antibody-bonding carrier was sufficiently washed with 0.02 M phosphate buffer solution containing 0.5 M NaCℓ. The washed antibody-bonding carrier contained 29.5 mg of anti-hM-CSF antibody per g of the antibody-bonded carrier.

Human urine concentrate, which was prepared from 1000 ℓ of human urine by concentration and dimineralization with an ultrafiltration concentrator, was adsorped to DEAE-cellulose to remove unadsorbed contaminants, then eluted with 0.3 M NaCℓ solution. To the eluate, NaCℓ was added to prepare a solution containing hM-CSF and 0.5 M NaCℓ. The specific activity of the resultant hM-CSF solution was $2 \times 10^5$ U/mg.

To 100 g of the previously prepared antibody-bonded carrier, the previously prepared hM-CSF solution was added to make 500 mℓ of the mixture in total quantity. The resultant mixture was stirred at a temperature equal to or lower than 10 °C over one night, then subjected to batchwise chromatography. The antibody-bonded carrier capturing hM-CSF was collected by filtration through a glass filter under aspiration and washed with 0.02 M phosphate buffer solution containg 0.5 M NaCℓ for three times. To the washed antibody-bonded carrier, 500 mℓ of 0.2 M acetate buffer solution (pH 2.5) was added, then stirred at 10 °C for 1 hour to elute hM-CSF. The resulted eluate was adjusted to a pH of 7.0, then concentrated and dimineralized by an ultrafiltration membrane to therby obtain the hM-CSF fraction. The resultant fraction was subjected to RP-HPLC using Hi-Pour 214Tp (by Biduck, 2.2cm diameter × 25 cm hight) with a 0 - 100 % linear-density gradient of acetonitrile containing 0.1 % trifluoroacetic acid to collect purified hM-CSF solution. The resultant solution was freeze-dried to obtain 3.2 mg purified hM-CSF. The specific activity of the purified hM-CSF was $1.4 \times 10^4$ U/mg, and its purity was >96% by SDS-PAGE method.

[PREPARATION OF HUMAN M-CSF, #3]
(cf: Japanese Unexamined Patent Application Gazette No.63(1988)-250400)

Anti-hM-CSF antibody, which was obtained from rabbits immunized with purified hM-CSF, was dyalized against 0.1 M phosphate buffer solution (pH 7.0) to thereby obtain a solution containing the antibody in the concentration of 20 mg/mℓ. To 100 g of Formyl Cellulofine® (trademark, by Chisso Manuf.), which had been previously washed with distilled water and 0.1 M phosphate buffer solution, 200 mℓ of the antibody solution was added and stirred for 2 hours at room temperature for adsorption. Then 700 mg of sodium cyanoboron hydride was further added there to and stirred for 16 hours to prepare antibody-bonded carrier. The resultant antibody-bonded carrier was washed with 0.2 M tris-HCℓ buffer solution, then 200 mℓ of the tris buffer solution containing 500 mg of sodium cyanoboron hydride was further added and stirred for 4 hours at room temperature to deactivate unreacted formyl groups. The resulted antibody-bonded carrier was sufficiently washed with 0.02 M phosphate buffer solution containing 0.5 M NaCℓ. The washed antibody-bonded carrier contained 29.5 mg of anti-hM-CSF antibody per g antibody-bonded carrier.

Human urine concentrate, which was prepared from 1000 mℓ of human urine by concentration and dimineralization with an ultrafiltration concentrator, was introduced into a DEAE-cellulose column to remove unadsorbable contaminants, then eluted with 0.3 M NaCℓ solution. To the resultant eluate, NaCℓ was added to prepare a solution containing hM-CSF and 0.5 M NaCℓ. The specific activity of the hM-CSF solution was $2 \times 10^5$ U/mg.

To 100 g of the previously prepared antibody-bonded carrier, the previously prepared hM-CSF solution was added to obtain 500 mℓ of the mixture in total quantity. The resultant mixture was stirred at a temperature equal to or lower than 10 °C over one night, then subjected to batchwise chromatography. The antibody-bonded carrier capturing hM-CSF was collected by filtration through a glass filter under aspiration and washed with 0.02 M phosphate buffer solution containg 0.5 M NaCℓ for three times. To the washed carrier, 500 mℓ of 0.2 M acetate buffer solution (pH 2.5) was added, then stirred at 10 °C for 1 hour to elute hM-CSF. After adjusting the pH of the resultant eluate to pH 7.0, it was subjected to concentration and dimineralization by an ultrafiltration membrane to thereby obtain 10 mg of purified hM-CSF. The specific activity of the purified hM-CSF was $5.2 \times 10^7$ U/mg, and the purity was >90 % by SDS-PAGE method.

[PREPARATION OF rhM-CSF]

Antibody-bonded carrier of rhM-CSF was prepared, washed, and then deactivated the unreacted formyl groups in the same manner as in [PREPARATION OF HUMAN M-CSF, #2]. The resulted antibody-bonded carrier was fully washed with 0.02 M phosphate buffer solution containing 0.5 M NaCℓ. The washed carrier contained 32.6 mg of anti-rhM-CSF antibody per g thereof.

Ten ℓ of a culture broth, produced by incubation of mammalian cells transfected with the hM-CSF producing gene (CHO cells) in a culture medium, was conentrated and demineralized by an ultrafiltration concentrator to thereby obtain its concentrate. The resulted concentrate was adsorbed to a DEAE-cellulose column to remove unadsorbed contaminants, then subjected to elution with 0.3M NaCℓ solution. To the eluate, NaCℓ was added to obtain a hM-CSF solution containing 0.5 M NaCℓ. The specific activity of the resultant hM-CSF solution was $3 \times 10^6$ U/mg.

To 100 g of the previously prepared antibody-bonded carrier, said hM-CSF solution was added to obtain 500 mℓ of the mixture in total quantity The resulting mixture was stirred at a temperature equal to or lower than 10 °C over one night, then subjected to batchwise chromatography. The antibody-bonded carrier capturing rhM-CSF was collected by filtration through a glass filter under aspiration and washed with 0.02 M phosphate buffer solution containing 0.5 M NaCℓ three times. To the washed antibody-bonded carrier capturing rhM-CSF, 500 mℓ of 0.2 M acetate buffer solution (pH 2.5) was added and stirred at 10 °C for 1 hour to elute rhM-CSF. The resultant eluate was adjusted to a pH of 7.0, then concentrated and demineralized by ultrafiltration membrane to thereby obtain a rhM-CSF fraction. This fraction was subjected to RP-HPLC using Hi-Pour 214TP column (by Biduck, 2.2 cm diameter × 25 cm hight) with a 0-100% linear-concentration gradient of acetonitrile (pH 2.0) containing 0.1 trifluoroacetic acid, to thereby obtain purified rhM-CSF solution. The purified solution was freeze-dried to obtain 25 mg of purified rhM-CSF. The specific activity of the resultant rhM-CSF was $1.9 \times 10^8$ U/mg, and its purity was >98% by SDS-PAGE method.

The physico-chemical nature of the hM-CSF obtained by any of the forgoing methods were as follows:

a) MOLECULAR WEIGHT

The obtained hM-CSF was a homo-dimer consisting of equivalent or homologous subunits and its molecular weight is 70,000 - 90,000 daltons measured by sodium dodecylsulfate polyacrylamide gel electrophoresis. The molecular weight of the subunit which was obtained by dissociation of the dimer with reducing agents to deactivate its biological activity was 35,000 - 45,000 daltons measured by the same method.

b) AMINO ACID SEQUENCE OF THE SUBUNIT PROTEIN OF hM-CSF

The subunit protein of the homodimer of hM-CSF isolated from human urine has a 214-238 amino acid sequence as shown hereunder and the asparagin located at 122nd and 140th had typical N-glycoside bondings represented by "asparagine (Asn) - x - threonine (Thr)/ serine (Ser)", wherein x denotes an optional amino acid. Just for reference, the subunit protein of the homodimer of rhM-CSF has the 223 amino acid sequence as shown hereunder.

Glu-Glu-Val-Ser-Glu-Tyr-Cys-Ser-His-Met-
Ile-Gly-Ser-Gly-His-Leu-Gln-Ser-Leu-Gln-
Arg-Leu-Ile-Asp-Ser-Gln-Met-Glu-Thr-Ser-
Cys-Gln-Ile-Thr-Phe-Glu-Phe-Val-Asp-Gln-
Glu-Gln-Leu-Lys-Asp-Pro-Val-Cys-Tyr-Leu-
Lys-Lys-Ala-Phe-Leu-Leu-Val-Gln-Asp-Ile-
Met-Glu-Asp-Thr-Met-Arg-Phe-Arg-Asp-Asn-
Thr-Pro-Asn-Ala-Ile-Ala-Ile-Val-Gln-Leu-
Gln-Glu-Leu-Ser-Leu-Arg-Leu-Lys-Ser-Cys-
Phe-Thr-Lys-Asp-Tyr-Glu-Glu-His-Asp-Lys-
Ala-Cys-Val-Arg-Thr-Phe-Tyr-Glu-Thr-Pro-
Leu-Gln-Leu-Leu-Glu-Lys-Val-Lys-Asn-Val-

Phe-Asn-Glu-Thr-Lys-Asn-Leu-Leu-Asp-Lys-
Asp-Trp-Asn-Ile-Phe-Ser-Lys-Asn-Cys-Asn-
Asn-Ser-Phe-Ala-Glu-Cys-Ser-Ser-Gln-Asp-
Val-Val-Thr-Lys-Pro-Asp-Cys-Asn-Cys-Leu-
Tyr-Pro-Lys-Ala-Ile-Pro-Ser-Ser-Asp-Pro-
Ala-Ser-Val-Ser-Pro-His-Gln-Pro-Leu-Ala-
Pro-Ser-Met-Ala-Pro-Val-Ala-Gly-Leu-Thr-
Trp-Glu-Asp-Ser-Glu-Gly-Thr-Glu-Gly-Ser-
Ser-Leu-Leu-Pro-Gly-Glu-Gln-Pro-Leu-His-

Thr-Val-Asp-Pro-Gly-Ser-Ala-Lys-Gln-Arg-

Pro-Pro-Arg-Ser-Thr-Cys-Gln-Ser-Phe-Glu-

Pro-Pro-Glu-Thr-Pro-Val-Val-Lys-

## c) ISOELECTRIC POINT

The isoelectric point (pI) of the homo dimer measured by isoelectric point electrophoresis of polyacrylamide gel and by sucrose-density-gradient isoelectric point electrophoresis fell within the range of 3.1 - 3.7.

## d) CIRCULAR DICHROISM SPECTRUM

Minimum peaks at 208 and 222 nm (wave length) were observed in the far-ultraviolet ray CD spectrum of the homo dimer measured by a circular dichroism polarimeter, and it was suggested to have an $\alpha$-helix

structure.

e) THERMAL STABILITY

The homo dimer did not lose its biological activity when heated at 60 ± 0.5 °C for 60 minutes.

f) INFRARED ABSORPTION SPECTRUM

Intense absorption was observed at 1680 cm$^{-1}$ and 1130 cm$^{-1}$ (wave length) and medium intense absorption was observed at 1540 cm$^{-1}$, 1430 cm$^{-1}$ and 1070 cm$^{-1}$ in the infrared absorption spectrum.

The hM-CSF prepared in accordance with the foregoing methods can be parenterally dosed (for example, intravenously, arteryally, intramascularlly, subcutaneously and intraabdominally). Injections and infusions containing hM-CSF in accordance with the present invention can be prepared by any conventional methods. For example, hM-CSF prepared in accordance with the present invention can be added to a preferable buffer solution, the resultant solution can be subjected to sterile membrane filtration, the resultant filtrate can be aseptically filled in vials then sealed to provide such products for injections and infusions. required, the filtrate can be freezedried.

It should be noted, however, that hM-CSF has the property to adhere onto surfaces of glass, plastics and sterile membrane filters. This adhesivness can be eliminated by adding such additives as, for example, surface active agents, human serum albumin and/or gelatin, It has been found that inclusion of surface active agents and/or proteins such as Tween 80, human serum albumin and gelatine may remarkably improve the biological activities of hM-CSF. Preferable concentrations of the additives are: more than 0.2 μg/mℓ for surface active agents; more than 1 mg/mℓ of hM-CSF preparation, or 100 fold weight part of human serum albumine or gelatin to hM-CSF.

The present invention will be described in further detail with reference to the accompanying drawings in which:

Fig. 1 is a graph showing myeloblast and leukocyte counts in peripheral blood of patients suffering from MDS after dosage of an hM-CSF preparation of the invention,

Fig. 2 is a graph showing normal leukocyte, normal neutrophil leukocyte and normal erythrocyte counts in a patient suffering from pediatric MDS after dosage of an hM-CSF preparation of the invention,

Fig. 3 is a graph showing efficacy of an hM-CSF preparation on tumoricidal activity of the human monocyte,

Fig. 4 is a graph showing efficacy of an hM-CSF preparation on antibody-dependent tumoricidal activity of human monocyte,

In accordance with the present invention, it has been confirmed that dosage of hM-CSF inclusive of human urinary M-CSF and rhM-CSF to patients suffering from MDS is effective not only to decrease myeloblast count in peripheral blood and to decrease the proportion of myeloblasts to total bone narrow cells, but also is effective to increase erythrocyte, leukocyte and neutrophil leukocyte counts in peripheral blood of the patients.

In accordance with the present invention, it is also confirmed that human urinary M-CSF is effective to significantly augment the tumoricidal activity of human monocytes and to inhibit proliferation of human cervical cancer cells. Therefore, human urinary M-CSF is effective in therapy against various malignant tumors on the hematopoietic organs and various organs, for example, myelogeneous leukemia and uterine cancer.

It is also confirmed that hM-CSF may significantly augment antibody dependent cell-mediated cytotoxicity (ADCC) of human monocytes. Therefore, hM-CSF is effective, when used in conjunction with a specific anti-malignant tumor antibody, to augment the therapeutic effect expected from single use of hM-CSF.

Dosage of hM-CSF varies depending upon the ages and conditions of the patients, typical dosages are as follows:

(1) AGAINST MYELODISPLASTIC SYNDROME

0.4 μg - 16 μg/kg (body weight)/day, preferablly
1.6 μg - 8 μg/kg (body weight)/day

9

(2) AGAINST MALIGNANT TUMORS

0.4 $\mu$g - 16 $\mu$g/kg/day [4 $\times$ 10$^4$ - 160 $\times$ 10$^4$ U/kg (body weight)/day], preferablly
1.6 $\mu$g - 8 $\mu$g/kg/day [16 $\times$ 10$^4$ - 80 $\times$ 10$^4$ U/kg (body weight)/day]

Now some examples of the present invention will be described hereunder for a better understanding of the present invention.

Example 1 [THERAPEUTIC EFFECTS AGAINST MYELODISPLASTIC SYNDROME]

(1) PREPARATION OF hM-CSF FOR MDS TREATMENT IN ACCORDANCE WITH THE PRESENT INVENTION

There were added different quantities of stabilizing agents as shown in Table 1 and hM-CSF (prepared in accordance with aforementioned [PREPARATION OF HUMAN M-CSF, #2]) to 20 mM phosphate buffer solution (pH 7.2). Solutions for test samples and control samples were prepared. The concentration of hM-CSF in all the solutions was 100 $\mu$g/m$\ell$. Each of the solutions was sterilized through a nitrocellulose membrane filter, 1 m$\ell$ aliquot of the resultant filtrate was aseptically dispensed into a vial, freeze-dried, and then sealed air-tight to thereby obtain a plurality of products for each sample.

(2) STABILITY OF BIOLOGICAL ACTIVITY OF THE SAMPLES

The stability and biological activity of the samples was measured by a conventional soft-agar-culture method using murine bone marrow cells. The results are also shown in Table 1.

As will be seen from Table 1, biological activities, after preservation at 40 ° C for 3 months, in those samples containing the surface active agent (Tween® 80, trademark) in a concentration equal to or more than 10 $\mu$g/m$\ell$, or containing human serum albumin or gelatine in a concentration equal to or more than 1 mg/m$\ell$ were maintained at 70 % or more of those immediately after preparation of the samples.

Table 1

| stabilizing agent & concentration (mg/m$\ell$) | | biological acitivity (%) | |
|---|---|---|---|
| | | after 1 month at 40 ° C | after 3 months at 40 ° C |
| Tween® 80 | 0.001 | 73 | 40 |
| | 0.010 | 93 | 74 |
| | 0.100 | 85 | 89 |
| human seru albumine | 0.5 | 69 | 49 |
| | 1.0 | 87 | 71 |
| | 5.0 | 89 | 93 |
| | 10.0 | 97 | 98 |
| gelatin | 0.5 | 63 | 36 |
| | 1.0 | 81 | 70 |
| | 5.0 | 81 | 77 |
| | 10.0 | 92 | 87 |
| nil | - | 43 | 18 |

(3) THERAPEUTIC EFFECT ON MYELODISPLASTIC SYNDROME

The therapeutic effect of the sample on a patient suffering from MDS was evaluated by measurement of changes in hematological parameteres, such as myeloblast, normal leukocyte, normal erythrocyte counts in the peripheral blood of the patient.

10

The preparation in accordance with the present invention, which was prepared in the same manner as described in (1) of Example 1 and was dissolved in physiological saline solution, was daily injected to a 40 year old patent by intravenous drip infusion for 14 consecutive days at the dosage of 1.6 μg of hM-CSF/kg/day.

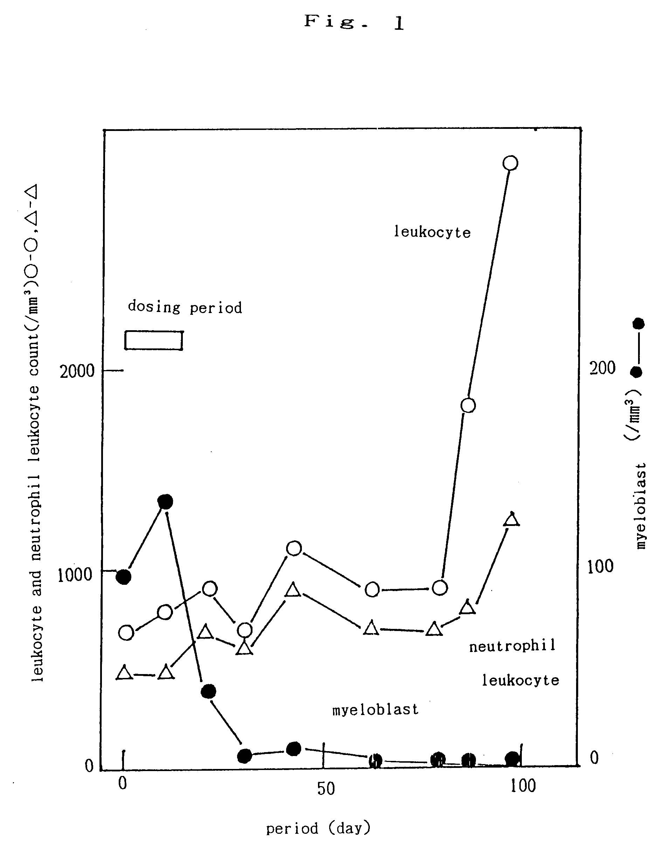

After initiation of infusion, myeloblast and leukocyte counts in peripheral blood of the patient were periodically measured. The results are shown in Fig. 1, wherein the abscissa denotes the period shown by days, the ordinate denotes respectively peripheral blood myeloblast count with ● - ● (right ordinate), and peripheral blood normal leukocyte count with ○ - ○ , and normal neutrophil leukocyte count with △ - △ (left ordinate) respectively.

As will be seen from Fig. 1, myeloblast count (● - ●) in the peripheral blood before initiation of infusion was 100/mm$^3$, it was decreased to 40/mm$^3$ on the 10th day, and to 10/mm$^3$ on the 30th day after initiation of infusion. Observation was continued until the 100th day after initiation of infusion, and it was confirmed that myeloblast count was kept at 0/mm$^3$.

Meanwhile, normal leukocyte (○ - ○ ) and neutrophil leukocyte (△ -△) counts in the peripheral blood before initiation of infusion were 700/mm$^3$ and 500/mm$^3$ respectively which was an abnormally decreased state of leukocyte count, but they were increased, after initiation of dosage, to 750/mm$^3$ and 500/mm$^3$ on the day 10 respectively, to 900/mm$^3$ and 600/mm$^3$ on day 30 respectively, and on day 90 they recovered almost their normal values, 1800/mm$^3$ and 900/mm$^3$ respectively.

It will be understood that the preparation in accordance with the present invention is effective against MDS.

Example 2 [THERAPEUTIC EFFECT ON PEDIATRIC MDS]

A clinical study was conducted on therapeutic effects of the preparation used in Example 1 on pediatric MDS.

To a 3 years old patient suffering from MDS whose parents gave their informed consent, the same preparation as used in Example 1 was daily injected by intravenous drip infusion for 9 consecutive days at the dosage of 2.4 μg of hM-CSF/kg/day as the effective component.

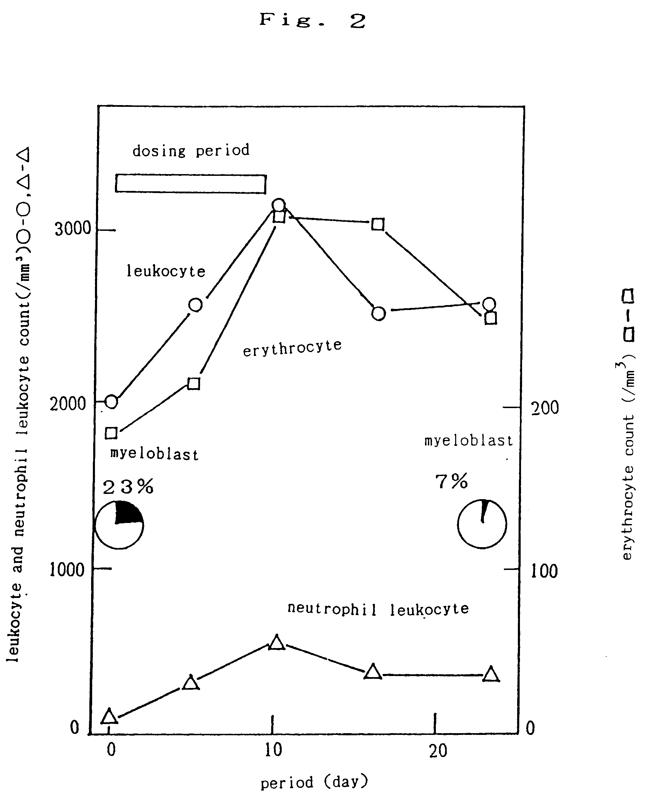

Normal leukocyte, normal neutrophil leukocyte and normal erythrocyte counts in the peripheral blood after initiation of infusion and the ratio of myeloblasts to total bone marrow cells before and after initiation of infusion were periodically measured. The results are shown in Fig. 2, wherein the abscissa denotes the period shown by days, the ordinates denote respectively peripheral blood leukocyte count with ○ - ○ , peripheral blood normal neutrophil leukocyte count with △ - △ (left ordinate), and peripheral blood erythrocyte count with □ - □ (right ordinate).

As will be seen from Fig. 2, normal erythrocyte count (□ - □) in the peripheral blood before initiation of infusion was 184 × 10$^4$/mm$^3$, normal leukocyte count (○ - ○ ) was 2000/mm$^3$ and normal neutrophil leukocyte count (△ - △) was 180/mm$^3$ respectively, they were increased, after initiation of infusion, to 208 × 10$^4$/mm$^3$ (normal erythrocytes), 2600/mm$^3$ (normal leukocytes) and 290/mm$^3$ (neutrophil leukocytes) on the 5th day, and on the 10th day they were increased to 308 × 10$^4$ /mm$^3$ (normal erythrocytes), 3100/mm$^3$ (normal leukocytes) and 530/mm$^3$ (normal neutrophil leukocytes). The ratio of myeloblast count to total bone marrow cells before initiation of infusion was 23 % as shown by segmental graph on the left side of Fig. 2, and after initiation of infusion it was remarkably decreased to 7 % as shown on the right side in Fig. 2 respectively.

It will be understood that the preparation in accordance with the present invention is effective against MDS.

Example 3 [THERAPEUTIC EFFECT ON MALIGNANT TUMORS]

Human urinary M-CSF prepared in accordance with the aforementioned [PREPARATION OF HUMAN M-CSF, #1] was used in this example. The hM-CSF was dissolved in 20 mM phosphate buffer solution (pH 7.2) together with different quantities of proteins as shown in Table 2 to prepare solutions of hM-CSF in the same concentration of 10 μg/mℓ for test and control samples. Each of the resultant solutions was sterilized through a nitrocellulose membrane filter, 1 mℓ aliquot of the resultant filtrate was aseptically dispensed into a vial, and then sealed air-tight without lyophilization thereby obtaining a plurality of products for each sample.

The stability of human urinary M-CSF activity and the function of hM-CSF to augment tumoricidal activity of human monocytes were studied using the resultant products.

The stability of hM-CSF preparation containing human urinary M-CSF was evaluated by measuring its biological activity in a conventional soft-agar-culture method using murine bone marrow cells. Studies on tumoricidal activity with respect to human monocytes were conducted using those samples which were stable for a long time in the above mentioned stability test.

The results of the stability test are shown in Table 2. As will be seen from Table 2, biological activity, after preservation for 3 months from their preparation at 4 °C, in those samples in which 1 mg/mℓ or more of human serum albumine or gelatine was added were kept at 70 % or more of those immediately after preparation of the samples. That is, the activity of those samples in which 1 mg/mℓ or more of human serum albumin or gelatine was added were stable and available for clinical use.

Table 2

| protein & quantity added (mg/mℓ) | | biological activity (%) | |
|---|---|---|---|
| | | 1 month | 3 months |
| | (mg/mℓ) | at 4 ℃ | at 4 ℃ |
| human serum albumin | 0.5 | 85 | 68 |
| | 1.0 | 90 | 95 |
| | 2.5 | 98 | 97 |
| | 5.0 | 98 | 99 |
| | 10.0 | 99 | 98 |
| gelatine | 0.5 | 81 | 65 |
| | 1.0 | 91 | 80 |
| | 2.5 | 92 | 85 |
| | 5.0 | 91 | 85 |
| nil | – | 75 | 55 |

Accordingly, it will be understood that when proteins were added more than 100 folds (weight) to human urinary M-CSF, stability of the biological activity in liquid state was improved.

The influence of said anti-tumor preparations, which was added with 5.0 mg/mℓ of human serum albumin as a stabilizer, to tumoricidal activity of human monocytes was measured by the method described hereunder. Human monocytes were harvested from peripheral blood of healthy men by means of Ficoll centrifugation, and a plastic adhesion method (monocyte population was more than 90 % of total cells). The isolated monocytes were suspended in RPMI 1640 medium ($10^5$/mℓ) containing 20 % of fetal calf serum, and added with the anti-tumor preparation in different concentrations of hM-CSF at 0 ng/mℓ (control), 25 ng/mℓ, 50 ng/mℓ and 150 ng/mℓ. The mixtures were incubated at 37 °C for two days, then human monocytes were washed with RPMI 1640 medium and collected. A 0.1 mℓ aliquot of the resultant suspension containing $10^6$/mℓ of the monocytes was inoculated into each well of microplate having 96 wells, and then a suspension of human myelogeneous leukemia cells (K-562 cells), labeled with $^{51}$Cr, in the concentration of $10^5$/mℓ was inoculated in the quantity of $^{100}\mu\ell$ per well. After incubation at 37 °C for 12 hours, the radioactivity of free $^{51}$Cr in supernatant of each well was measured and then the tumoricidal activity was calculated therefrom in accordance with the following formula.

$$\frac{\text{activity of free } ^{51}\text{Cr}}{\text{activity of total } ^{51}\text{Cr}} \times 100 = \text{tumoricidal activity (\%)}$$

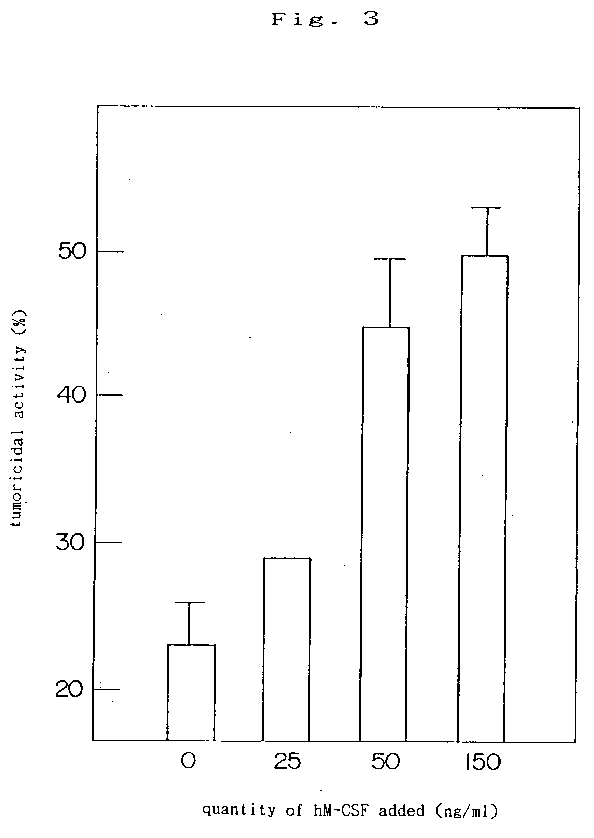

The results are shown in Fig 3. As will be seen from Fig. 3, monocytes incubated under the presence of the anti-tumor preparation showed a significantly augmented tumoricidal activity of monocytes than those incubated under absence of anti-tumor preparation. It was found that the effect of hM-CSF on the augmentation of tumoricidal activity of monocytes shows a dose-response manner.

12

In Fig. 3, mean values and standard deviations of the results of the tests, which were concurrently conducted, are shown wherein the value of 100 means complete killing of tumor cells.

Example 4 [EFFICACY OF THE PREPARATION ON ANTIBODY-DEPENDENT CELL-MEDIATED CYTOTOXICITY OF HUMAN MONOCYTES]

A plurality of solutions wherein different quantities of proteins as shown in Table 3 with 10 $\mu$g/m$\ell$ of human urinary M-CSF included therein were prepared in the same manner as in Example 3. Each of the solution was subjected to sterile membrane filtration, the resultant filtrate was dispensed into vials by 1 m$\ell$ each, and freezedried, then sealed to prepare anti-tumor preparations (test samples). Evaluation on the preservation stability of hM-CSF activity and of the augmentation effects of anticancer preparations on antibody-dependent cell-mediated cytotoxicity (ADCC) of human monocytes were carried out.

The stability was measured by a soft-agar-culture method using murine bone marrow cells as in the same manner in Example 1.

The studies on the augmentation efficacy upon ADCC of human monocytes were conducted only on those preparations which were stable in the preservation test.

The results are shown in Table 3. As will be seen from Table 3, the biological activity of the preparations in which 1 mg/m$\ell$ or more of human serum albumin or gelatine were added was kept at more than 70% even after preservation at 40°C for 3 months, and found to be stable.

In other words, it was confirmed that stability can be provided when proteins are added more than 100 fold to hM-CSF.

Table 3

| protein | | biological activity (%) | |
|---|---|---|---|
| protein added | quantity added (mg/m$\ell$) | 1 month at 40 °C | 3 months at 40 °C |
| human serum albumin | 0.5 | 73 | 55 |
| | 1.0 | 80 | 73 |
| | 2.5 | 88 | 90 |
| | 5.0 | 98 | 85 |
| | 10.0 | 99 | 92 |
| gelatine | 0.5 | 71 | 45 |
| | 1.0 | 80 | 70 |
| | 2.5 | 85 | 70 |
| | 5.0 | 81 | 82 |
| | 10.0 | 92 | 87 |
| nil | - | 45 | 20 |

The efficacy of the preparations on ADCC on human monocytes was studied in the manner described hereunder:

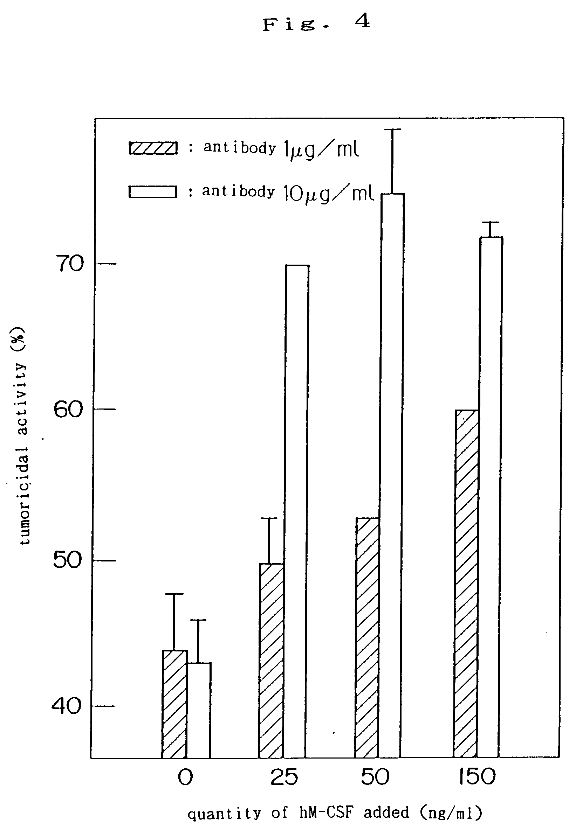

The anticancer preparations including 5.0 mg/m$\ell$ of gelatine in Table 3 were used. As in the same manner in Example 3, anti-tumor preparations were respectively added to human monocytes, the resulted solutions were incubated for 2 days in the respective culture medium, then washed to collect human monocytes. Into each well of a 96 well microplate, a monocytes suspension in $10^6$/m$\ell$ concentration was inoculated 100 $\mu\ell$ each. Into each well, Raji cells originating from human lymphoma and the anti-Raji cell specific antibody were respectively inoculated in the concentration of 0 $\mu$g/m$\ell$ (control), 1 $\mu$g/m$\ell$ or 10 $\mu$g/m$\ell$ and incubated respectively. After a 12 hours incubation, the respective supernatant were measued for free $^{51}$Cr radioactivity, and the tumoricidal activity thereof calculated in the same manner as in Example 3. The results are shown in Fig. 4.

As will be seen from Fig. 4, a significant augmentation of ADCC was observed for the monocytes which were incubated under the presence of anti-tumor preparations as well as anti-tumor antibody in comparison

13

with those under absence thereof. The augmentative efficay was linearly proportional to the concentarations of human urinary M-CSF, which is the effective component of the preparations, and the specific antibody.

In Fig. 4, the mean values and the standard deviations of the values, resulted from concurrently conducted experiments, are shown wherein the value of 100 means 100 % of tumor cells were killed.

Example 5 [EFFICACY OF ANTI-TUMOR PREPARATIONS AGAINST PROLIFERATION OF HUMAN CERVICAL CANCER CELLS]

BALB/C nude mice were divided into 3 groups (10 mice/group) and they were subcutaneously transplanted with $10^5$ of human cervical cancer cells (Hela cells) respectively. The anti-tumor preparation of Example 3 was intraperitoneally dosed to the mice every day for 7 consecutive days from the next day of the transplantation in the different dosage at 4.0 $\mu$g/kg body weight (40 × $10^4$ U/kg body weight), 16 $\mu$g/kg body weight (160 × $40^4$ U/kg), 64 $\mu$g/kg body weight (640 × $10^4$ U/kg). On the 28th day after the transplantation, the tumors were enucleated and weighed. Thereby the anti-tumor activities were measured. The results are shown in Table 4.

As will be seen from Table 4, minimization of tumors were apparently observed for the mice to which more than 16 $\mu$g/kg (160 × $10^4$ U/kg body weight) of anti-tumor preparations were dosed, and it was concluded that the preparations have anti-tumor activity.

Table 4

| dosage (× $\mu$g/kg) | total weight of tumors (mg) |
|---|---|
| 0 (control) | 725 ± 150 |
| 4.0 | 638 ± 180 |
| 16.0 | 480 ± 95 |
| 64.0 | 395 ± 110 |

Example 6 [EFFICACY OF HUMAN M-CSF ON PRESENTAION OF Fc RECEPTORS ON HUMAN MONOCYTES]

The human monocytes isolated in Example 3 were incubated under the presence of and under the absence of anti-tumor preparations respectively for 2 days. Each of the resultant cultures was mixed with sheep erythrocytes which were previously coated with rabbit IgG. The resultant mixtures wrere sufficiently washed, then the erythrocytes which combined with monocytes were counted for a judgement of presentation of Fc receptors. The results are shown in Table 5.

As will be seen from Table 5, apparently a large number of erythrocytes were combined to monocytes which were incubated under the presence of anti-tumor preparations.

From the results of Example 6, it was found that human urinary M-CSF augmented the ADCC activity of human monocytes. It is assumed that the results are derived from acceleration of Fc-receptor-presentaion on monocytes by the anti-tumor preparations and strengthened bonding of monocytes to the target tumor cells.

Table 5

| [EFFICACY OF HUMAN M-CSF IN PRESENTATION OF Fc RECEPTORS ON HUMAN MONOCYTES] | | | |
|---|---|---|---|
| human urinary M-CSF (ng/m$\ell$) | 0 | 25 | 50 |
| erythrocytes/monocytes | 1.5 ± 2.0 | 3.1 ± 2.4 | 5.0 ± 3.2 |

In Table 5, values are shown with the mean values and standard deviations of counted erythrocytes which combined with monocytes among 100 monocytes.

It will be apparent from the forgoing examples that the present invention provides therapeutic preparations against MDS which had been difficult to cure. The anti-MDS preparations of the present

EP 0 385 385 B1

invention can reduce or disperse myeloblasts and increase normal peripheral blood cells.

It will be understood that the present invention provides therapeutic preparations which have remarkable anticancer activity against specific cancers and which have no severe side effects. The anticancer preparations of the present invention are effective for therapy against specific malignant tumors by itself and are also effective thereto when they are dosed together with a specific antibody against the malignant tumors concerned.

**Claims**

1. Use of hM-CSF for the manufacture of a medicament for treating myelodysplastic syndrome and paediatric myelodysplastic syndrome in humans.

2. Use according to claim 1 wherein said treatment is effective for decreasing the myeloblast count in peripheral blood and for decreasing the proportion of myeloblast to total bone marrow cells while increasing erythrocyte, leukocyte and neutrophil leukocyte counts in peripheral blood in humans.

3. Use of hM-CSF in the manufacture of a medicament for the treatment of myelogenous leukemia in humans.

4. Use of hM-CSF in the manufacture of a medicament which is effective when combined with anti Raji cell specific antibody for the treatment of lymphoma in humans.

5. Use of hM-CSF in the manufacture of a medicament for the treatment of uterine cervix carcinoma in humans.

6. Use according to any one of claims 3-5 wherein said hM-CSF enhances the tumoricidal activity of human monocytes.

7. Use according to any one of claims 3-5 wherein said hM-CSF increases the number of Fc receptors to be presented on monocytes.

8. The uses according to any one of claims 1 to 7 wherein said hM-CSF is rhM-CSF.

9. The use according to any preceding claim wherein a therapeutically effective amount of said hM-CSF is used.

10. The use according to claim 9 wherein in the case of the uses mentioned in preceding claim 1 said therapeutically effective amount is 0.4-16$\mu$g/kg body weight/day.

11. The use according to claim 9 wherein in the case of the uses mentioned in claim 1 said therapeutically effective amount is 1.6-8$\mu$g/kg body weight/day.

12. The use according to any one of claims 3-5 and 8-11 wherein said medicament comprises an effective amount of hM-CSF and also an effective amount of antibody to the particular tumours.

**Patentansprüche**

1. Verwendung von hM-CSF zur Herstellung eines Medikaments zur Behandlung des myelodysplastischem Syndroms des pädiatrischen myelodysplastischen Syndroms bei Menschen.

2. Verwendung nach Anspruch 1, bei welcher die Behandlung zur Verminderung der Myeloblastenzahl im peripheren Blut und zur Verminderung des Anteils von Myeloblasten an den Gesamtzahl der Knochenmarkzellen bei gleichzeitiger Erhöhung der Zahl der Erythrozyten, Leukozyten und neutrophilen Leukozyten im peripheren Blut bei Menschen.

3. Verwendung von hM-CSF bei der Herstellung eines Medikaments für die Behandlung von myelogener Leukämie bei Menschen.

15

**4.** Verwendung von hM-CSF bei der Herstellung eines Medikaments, das zur Behandlung von Lymphom bei Menschen bei Vereinigung mit "Raji"-zellspezifischem-Antikörper wirksam ist.

**5.** Verwendung von hM-CSF bei der Herstellung eines Medikaments für die Behandlung der Zervikarzinomen der Gebärmutter bei Menschen.

**6.** Verwendung nach einem der Ansprüche 3 bis 5, bei welcher der hM-CSF die tumorizide Aktivität von Humanmonozyten verstärkt.

**7.** Verwendung nach einem der Ansprüche 3 bis 5, bei welcher der hM-CSF die Zahl der auf den Monozyten präsentierten Fc-Rezeptoren erhöht.

**8.** Verwendung nach einem der Ansprüche 1 bis 7, bei welcher der hM-CSF rhM-CSF ist.

**9.** Verwendung nach einem der vorgenannten Ansprüche, bei welcher eine therapeutisch wirksame Menge des hM-CSF verwendet wird.

**10.** Verwendung nach Anspruch 9, bei welcher im Fall der Verwendung nach Anspruch 1 die therapeutisch wirksame Menge 0,4 ... 16 $\mu$g/kg Körpergewicht und Tag beträgt.

**11.** Verwendung nach Anspruch 9, bei welcher im Fall der Verwendung nach Anspruch 1 die therapeutisch wirksame Menge 1,6 ... 8 $\mu$g/kg Körpergewicht und Tag beträgt.

**12.** Verwendung nach einem der Ansprüche 3 bis 5 und 8 bis 11, bei welcher das Medikament eine wirksame Menge von hM-CSF umfaßt sowie ebenfalls eine wirksame Menge Antikörper der speziellen Tumore.

**Revendications**

**1.** Utilisation de hM-CSF pour la préparation d'un médicament destiné au traitement chez l'homme du syndrome de myélodysplasic et du syndrome de myélodysplasie pédiatrique.

**2.** Utilisation suivant la revendication 1, dans laquelle ledit traitement est efficace pour diminuer le nombre de myéloblastes dans le sang périphérique et pour diminuer la proportion de myéloblastes par rapport au nombre total de cellules de la moelle osseuse tout en augmentant le nombre d'érythrocytes, leucocytes et leucocytes neutrophiles dans le sang périphérique chez l'homme.

**3.** Utilisation hM-CSF pour la préparation d'un médicament destiné au traitement chez l'homme de la leucémie myélogène.

**4.** Utilisation de hM-CSF pour la préparation d'un médicament qui est efficace chez l'homme dans le traitement du lymphome quand il est associé à un anticorps spécifique anti cellule de Raji.

**5.** Utilisation de hM-CSF pour la préparation d'un médicament destiné au traitement chez l'homme du carcinome du col utérin.

**6.** Utilisation suivant l'une quelconque des revendications 3 à 5, dans laquelle ledit hM-CSF augmente l'activité antitumorale (en anglais : "tumoricidal") des monocytes humains.

**7.** Utilisation suivant l'une quelconque des revendications 3 à 5, dans laquelle ledit hM-CSF augmente le nombre de récepteurs Fc présents sur les monocytes.

**8.** Utilisation suivant l'une quelconque des revendications 1 à 7, dans laquelle ledit hM-CSF est rhM-CSF.

**9.** Utilisation suivant l'une quelconque des revendications précédentes, selon laquelle une quantité thérapeutiquement efficace dudit hM-CSF est utilisée.

**10.** Utilisation suivant la revendication 9, dans laquelle, pour les indications mentionnées dans la revendication 1, ladite quantité thérapeutiquement efficace est de 0,4-16 $\mu$g/kg/j.

**11.** Utilisation suivant la revendication 9, dans laquelle, pour les indications mentionnées dans la revendication 1, ladite quantité thérapeutiquement efficace est de 1,6-8 $\mu$g/kg/j.

**12.** Utilisation suivant l'une quelconque des revendications 3-5 et 8-11, dans laquelle ledit médicament comprend une quantité efficace de hM-CSF ainsi qu'une quantité efficace d'un anticorps de tumeurs particulières.

Fig. 1

Fig. 2

Fig. 3

quantity of hM-CSF added (ng/ml)

EP 0 385 385 B1

Fig. 4

Legend:
- ▨ : antibody 1μg/ml
- ▢ : antibody 10μg/ml

y-axis: tumoricidal activity (%)

x-axis: quantity of hM-CSF added (ng/ml)
values: 0, 25, 50, 150

21